# EUROPEAN PATENT SPECIFICATION

(11) **EP 1 427 681 B1**
(45) Date of publication and mention of the grant of the patent: **28.04.2010**
(21) Application number: 02777121.1
(22) Date of filing: 12.09.2002
(51) Int. Cl.: C04B 24/32, C04B 24/02, C04B 24/04, C04B 24/16, C07C 43/11, C07C 43/205, C07C 305/04, C07C 53/126, C07C 31/125

(54) **Water-reducing admixture comprising WORKABILITY-IMPROVING AGENT FOR CEMENT COMPOSITIONS**
Wasserreduktionszusatzmittel umfassend VERARBEITBARKEITSMITTEL FÜR ZEMENTZUSAMMENSETZUNGEN
Adjuvant de Reducteur d'eau comprenant un AGENT D'AMELIORATION DE LA MANIABILITE DESTINES A DES COMPOSITIONS DE CIMENT

(30) Priority: 19.09.2001 JP 2001285658; 07.05.2002 JP 2002131147
(43) Date of publication of application: 16.06.2004
(73) Proprietor: Construction Research & Technology GmbH, 83308 Trostberg (DE)
(72) Inventor: YAGUCHI, Minoru, Hagizono, Chigasaki-shi, , Kanagawa 2722 (JP); MATSUMOTO, Toshimi, Hagizono, Chigasaki-shi, Kanagawa 2722 (JP); UMEZAWA, Kenichi, Hagizono, Chigasaki-shi, Kanagawa 2722 (JP)
(86) International application number: PCT/EP2002/010390
(87) International publication number: WO 2003/024885

(56) References cited:
- EP-A- 0 238 858
- EP-A- 0 640 384
- WO-A-01/74733
- WO-A-95/16515
- GB-A- 2 343 447
- US-A- 4 351 671
- US-A- 5 679 150
- CHEMICAL ABSTRACTS, vol. 109, no. 26, 26 December 1988 (1988-12-26) Columbus, Ohio, US; abstract no. 235951u, TOMIZAWA ET AL: "Cement compositions for polymer concrete with good workability and bonding strength" XP000157957 & JP 63 176345 A (SUMITOMO CHEMICAL) 20 July 1988 (1988-07-20)
- DATABASE WPI Section Ch, Week 199511 Derwent Publications Ltd., London, GB; Class A93, AN 1995-077897 XP002227511 & JP 07 002557 A (NIPPON CEMENT KK), 6 January 1995 (1995-01-06)

## Description

The present invention relates to a workability-improving agent used for improving workability when producing cementitious compositions such as concrete and mortar, and further relates to a water-reducing admixture for cement comprising said workability-improving agent.

When producing cementitious compositions such as concrete and mortar, the most economic mixture is selected from the viewpoint of design strength, fluidity, workability, durability etc. However, since natural materials are used, it is not possible to avoid a variation in parameters such as particle diameter and particle size distribution. As a result of these variations, workability also varies, and when workability decreases, there occur problems, such as an increase in the risks involved in the work process and cracking of the concrete. For example, in mixtures with a low unit cement content, there is often a strong tendency for the workability of concrete to decrease considerably, as a result of these variations.

In such a case, it is possible to adjust the particle size distribution of the aggregate to a suitable range to improve workability. However, this makes the work more complicated and time is required for the adjustment.

It is also possible to improve workability by increasing the unit cement content. However, again this is inefficient, because of the time required to adjust the mixture, and the increase in cement content increases the cost.

It has now been found that it is possible to provide a workability-improving agent that substantially or completely overcomes these problems. The invention therefore provides a water-reducing-admixture, comprising a workability-improving agent for improving the workability of cement compositions comprising polyalkylene oxide derivatives represented by the formula (1):

R¹-(CO)ₐ-(O)_{b}-(AO)ₙ-X (1)

wherein R¹ is a hydrocarbon group of from 3 to 18 carbon atoms or a residual group of a polyamine derivative; a and b are 0 or 1; AO is an alkylene oxide of from 2 to 3 carbon atoms; (AO)ₙ is a homopolymer or a block or random copolymer of alkylene oxide units of from 2 to 3 carbon atoms, n is an integer of from 1 to 30; X is H, methyl, ethyl, propyl, butyl or SO₃Y¹, where Y¹ is H, Na and NH₄; and a hydrocarbon derivative of formula (2):

R²-(CO)_{c}-OH (2)

wherein R² is a aliphatic hydrocarbon group of 8 to 18 carbon atoms, and c is an integer of 0 or 1, and wherein the mixing ratio of the polyalkylene oxide derivatives to the hydrocarbon derivatives is between 90/10 to 50/50, and at least one water-reducing agent selected from the group consisting of lignosulfonate-type water-reducing agents, polycarboxyate-type water-reducing agents, polyol-type water-reducing agents, naphthalenesulfonate-type water-reducing agents and melaminesulfonate-type water-reducing agents.

Under the given invention reference is made to the following prior art documents: Chemical Abstracts, vol. 109, no. 26, December 26, 1988, Columbus, Ohio, US (Abstract no.: 235951u, Tomizawa et.al: "Cement compositions for polymer concrete with good workability and bonding strength", XP 000157957) and GB-A-2 343 447 of Grace.

The Chemical Abstracts document discloses a cementitious composition comprising polyoxyethylenstyreneether as well as styryl alcohol. GB 2 343 447 discloses a composition containing an alkylacrylpolyetheralcohol for improving the freeze-thaw durability of concrete. In consequence the Chemical Abstracts document discloses specific polyalkylenoxyd derivatives as typical hydrocarbone derivatives, and GB 2 343 447 discloses water reducing agents.

The present invention further relates to the abovementioned workability-improving agent wherein the residual group of a polyamine derivative is at least one of the group consisting of polyalkylene amine, polyvinylamine, allylamine, polyomithine, polylysine and polyarginine.

The present invention also relates to the abovementioned workability-improving agent, wherein the polyalkylene amine is at least one of the group consisting of polyethyleneimine, poly(dicarboxyalkylene)polyethyleneimine, diethyleneamine, triethylenetetramine, tetraethylenepentamine, pentaethylenehexamine, dipropylenetriamine, tripropylenetetramine, tetrapropylenepentamine and poly(dicarboxyalkylene)polyamideamine.

The present invention also relates to the abovementioned workability-improving agent, wherein the weight proportion of ethylene oxides contained in the polyalkylene oxide derivatives is at least 12 %, preferably at least 40%.

The present invention also relates to a method of improving the workability of a fluid cementitious composition comprising water and cement, comprising the addition to the composition of a workability-improving agent as hereinabove described.

The present invention also relates to a fluid cementitious composition of improved workability, comprising cement, water and a workability-improving agent as hereinabove described.

The present invention further relates to the abovementioned water-reducing admixture wherein the mixing ratio of the workability-improving agent to the water-reducing agent lies between 5/95 and 70/30.

The present invention further relates to a method of reducing the water content required for the attainment of fluidity in a fluid cementitious composition, by the addition to a cementitious composition comprising cement and water of a water-reducing admixture as hereinabove defined.

According to Japanese Industrial Standard JIS A 0203 the workability of concrete is defined as "the quality of fresh concrete as shown by the ease with which work is undertaken through consistency and the level of resistance to the segregation of materials necessary for concrete of a uniform quality". In general, the grade, grain size and proportion of fine particles of the aggregate used in concrete have a big influence on workability. The present specification, based on this definition, particularly uses slumping and bleeding as indicators of workability.

While the mechanisms leading to the improvement of workability are not completely understood, it is believed that the tiny independent air bubbles produced in the concrete by an air-entraining agent function in the manner of ball bearings. Thus, it is believed that, under conditions of an identical amount of air, the working mechanism of the workability-improving agent according to the present invention increases this ball bearing effect by reducing the size of the tiny independent air bubbles entrained in the concrete.

A preferred workability-improving agent of the present invention comprises at least one polyalkylene oxide derivative (A) represented by the formula (1):

R¹-(CO)ₐ-(O)-(AO)ₙ-X (1)

wherein R¹ is a hydrocarbon group of 3 to 18 carbon atoms, a and b are 0 or 1; AO is alkylene oxide of 2 to 3 carbon atoms; (AO)ₙ is a homopolymer or a block or random copolymer of alkylene oxide units of from 2 to 3 carbon atoms; n is an integer of from 1 to 30; X is H, SO₃Y¹; Y¹ represents H, Na and NH_{4;} and hydrocarbon derivatives represented by the formula (2):

R²-(CO)_{c}-OH (2)

wherein R² is a an aliphatic hydrocarbon group of 8 to 18 carbon atoms, and c is an integer of 0 or 1.

The polyalkylene oxide derivatives (A) and the hydrocarbon derivatives (B) may each be used on their own as workability-improving agent, or both may be used at the same time.
When both (A) and (B) are used at the same time, a mixing ratio of (A) to (B) of 90/10 - 50/50 being particularly preferred.

Further, preferred examples of specific combinations of polyalkylene oxide derivatives (A) and hydrocarbon derivatives (B) include polyoxyethylene lauryl ether (EO=6) and octanol, polyoxyethylene oleate (EO=10) and decanol, polyoxyethylene tridecyl ether (EO=10) and oleynol, polyoxyethylene octyl phenyl ether (EO=12) and hexanol, polyoxyethylene 2-ethylhexyl ether (EO=9) and tridecyl alcohol, polyoxyethylene (EO=6) polyoxypropylene (PO=2)-2-ethylhexyl ether and lauryl alcohol, polyoxyethylene oleate (EO=8) and cetyl alcohol, polyoxyethylene-2-ethylhexyl ether (EO=6) and octanol, polyoxyethylene (EO=7) oxypropylene (PO=1) tridecyl ether and decanol, polyoxyethylene (EO=6) oxypropylene (PO=1)-2-ethylhexyl ether and oleate, 2-ethylhexyloxy polyoxyethylene (EO=10) sulfonic acid and octanol, oleylpolyoxyethylene (EO=25) sulfonate sodium and octanol, 2-ethylhexyloxy polyoxyethylene (EO=8) oxypropylene (PO=1) sulfonate ammonium and decanol, polyoxyethylene (EO=12) polyethyleneimine (n=35) adduct and decanol, polyoxyethylene (EO=10) triethylenetetramine adduct and octanol, polyoxyethylene (EO=9) polyoxypropylene (PO=2) poly(dicarboxyethylene) (n=2) polyamide (n=3) amine and octanol, polyoxyethylene (EO=2) poly(dicarboxyethylene) (n=2) polyamide (n=4) amine and octanol, polyoxyethylene (EO=25) polyomithine (n=4) and octanol, polyoxyethylene (EO=12) polyethyleneimine (n=35) adduct and decanol, polyoxyethylene (EO=10) polyethyleneimine (n=3) amine and octanol. The combinations polyoxyethylene-2-ethylhexyl ether (EO=6) and octanol, polyoxyethylene (EO=7) oxypropylene (PO=1) tridecyl ether and decanol, polyoxyethylene (EO=6) oxypropylene (PO=1)-2-ethylhexyl ether and octanol, 2-ethylhexyloxy polyoxyethylene (EO=10) sulfonic acid and octanol, oleylpolyoxyethylene (EO=25) sulfonate sodium and octanol, 2-ethylhexyloxy polyoxyethylene (EO=8) oxypropylene (PO=1) sulfonate ammonium and decanol, polyoxyethylene (EO=12) polyethyleneimine (n=35) adduct and decanol, polyoxyethylene (EO=10) polyethyleneimine (n=3) amine and octanol, polyoxyethylene (EO=2) poly(dicarboxyethylene) (n=2) polyamide (n=4) amine adduct and octanol are particularly preferred.

The polyalkylene oxide derivatives (A) have, in their molecules, a homopolymer or a copolymer of alkylene oxide units of 2 to 3 carbon atoms (i.e., ethylene oxide (represented by EO hereinafter) and/or propylene oxide (represented by PO hereinafter)). The copolymer may be a block polymer or a random polymer.

From the viewpoint of water solubility, it is preferable that the weight proportion of ethylene oxide contained in the polyalkylene oxide derivatives (A) be at least 12 %, even more preferably at least 40 % and most preferably at least 50 %.

The water-reducing admixture for cement of the present invention additionally includes a known water-reducing agent (C). Suitable water-reducing agents that can be used in the present invention are not particularly limited, but typical examples include lignosulfonate types, polycarboxylate types, particularly of the types described in JP-B-S59-18338, JP-A-S6I-31333, JP-A-H02-163108, JP-A-HOS-306152, JP-A-H06-64956 and Japanese Patent No. 3235002, polyol types, naphthalenesulfonate types and melaminesulfonate types. One or more such water-reducing agents can be used in combination.

Specific examples of lignosulfonate-type water-reducing agents include POZZOLITH (trade mark) No. 8 made by NMB Co., Ltd., POZZOLITH No. 70, POZZOLITH No. 70L, SANFLO (trade mark) KS made by Sanflo Paric Co., Ltd. and YAMASO (trade mark) 90 made by Yamaso Chemical Co., Ltd.

Specific examples of polycarboxylate-type water-reducing agents include RHEOBUILD (trade mark) SP8N made by NMB Co., Ltd., PARIC (trade mark) 300S made by Sanflo Paric Co., Ltd., CHUPOL (trade mark) HP-11 made by Takemoto Oil & Fats Co., Ltd. SIKAMENT (trade mark) 1100NT made by Nihon Sika Co., Ltd. and MIGHTY (trade mark) 3000S made by Kao Corp.

Specific examples of polyol-type water-reducing agents include POZZOLITH (trade mark) No. 12R and POZZOLITH No. 70L, both made by NMB Co., Ltd.
A specific example of a naphthalenesulfonate-type water-reducing agent is RHEOBUILD SP9N made by NMB Co., Ltd.

A specific example of a melaminesulfonate-type water-reducing agent is RHEOBUILD 4000 made by NMB Co., Ltd.

In the water-reducing admixture for cement of the present invention, it is preferable from the viewpoint of the workability-improving effect and of fluidity that the weight ratio of the workability-improving agent to the water-reducing agent (C) lie between 5/95 and 70/30, and especially preferable that it lies between 10/90 and 50/50.

According to the present invention, the amount of workability-improving agent and water-reducing agent for cement can be appropriately selected; basically it may be determined so as to obtain the desired workability-improving effect or the desired fluidity. It is preferable that the amount of polyalkylene oxide derivatives (A) represented by the abovementioned formula (1) and hydrocarbon derivatives (B) represented by the abovementioned formula (2) calculated as solid parts of the cement weight in the cementitious composition lies between 0.01 wt% and 5 wt%, and especially preferable that it lies between 0.1 wt% and 5 wt%.

The workability-improving agents and the water-reducing admixtures according to the present invention have versatility and can be mixed with other admixtures as required. Examples of other admixtures include commonly used air-entraining agents, polysaccharide derivatives, drying shrinkage reducing agents, accelerators, retarders, foaming agents, defoaming agents, rust preventing agents, quick setting agents, thickeners, water soluble high polymeric substances, etc.

The invention will now be further described by means of the following non-limiting examples.

In Table 1 below, the chemical structure of the polyalkylene oxide derivatives (A) in the present invention are given for samples A-1 to A-7 and A-9 to A-12. Moreover, the polyalkylene oxide derivative given as sample A-8 is used for the purpose of comparison.

**Table 1: Polyalkylene oxide derivatives**

| Sample | Chemical structure |
|---|---|
| A-1 | C₈H₁₇O(EO)₆(PO)₁H |
| A-2 | C₈H₁₇O(EO)₁₀SO₃H |
| A-3 | C₁₇H₃₃COO(EO)₂₅SO₃Na |
| A-4 | C₈H₁₇O(EO)₈(PO)₁SO₃NH₄ |
| A-5 | CH₃(CH₂)₁₆COO(EO)₈(PO)₁H |
| A-6 | 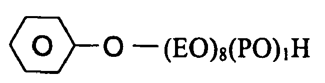 |
| A-7 | C₅H₁₁O(EO)₁(PO)₃H |
| A-8 | C₁₈H₃₇O(EO)₁₀₀H |
| A-9 | H(EI)₃₅(EO)₁₂H |
| A-10 | H₂N(EI)₃(EO)₁₀H |
| A-11 | H₂N(EI)_{2O}C(CH₂)₄CO(EI)₂-(PO)₂(EO)₉H |
| A-12 | C₁₃H₂₇O(EO)₇(PO)₁H |

| | |
|---|---|
| Note: In the table, EO represents ethylene oxide, PO represents propylene oxide and EI represents ethyleneimine. | |

In Table 2 below, the chemical structure of the hydrocarbon derivatives (B) in the present invention are given for samples B-1 to B-5. Moreover, the hydrocarbon derivatives given as sample B-6 is used for the purpose of comparison.

**Table 2: Hydrocarbon derivatives**

| Sample | Chemical structure |
|---|---|
| B-1 | C₈H₁₇OH |
| B-2 | C₁₀H₂₁OH |
| B-3 | C₁₂H₂₅OH |
| B-4 | C₁₈H₃₅OH |
| B-5 | CH₃(CH₂)₁₆COOH |
| B-6 | C₃H₇OH |

Concrete is prepared as shown below and the workability of the concrete is evaluated by visual observation in order to confirm the effect of the workability-improving agent of the present invention.

### (Workability evaluation)

Workability is evaluated by an experienced technician for cement compositions with equal slump and equal amount of entrained air by the manner in which slump occurred and by determining whether or not bleeding had occurred after the concrete had been left standing for 30 minutes. The evaluation is conducted in 4 grades: A (good), B (satisfactory), C (normal) and D (bad), as shown in Table 3.

**Table 3: Workability evaluation**

| Workability Evaluation | | Bleeding | | |
|---|---|---|---|---|
| | | none | little | much |
| Slump | none | A | B | C |
| | little | B | B | C |
| | much | C | C | D |

### (Composition and mixing of concrete)

Concrete with a target slump of 18.0 ± 1.0 cm and a target amount of entrained air of 4.5 ± 0.5 % is prepared with the mixtures shown in Table 4. The grade of the workability is determined by observing the workability of concrete prepared by weighing each material so as to obtain a mixed amount of 80 litres, introducing all the materials into a 100 litre pan-type forced mixer and mixing them for 120 seconds.

**Table 4: Concrete mixtures**

| Mix No. | Water/ cement ratio (%) | Fine aggregate ratio (%) | Unit amount (kg/m³) | | | |
|---|---|---|---|---|---|---|
| | | | water for mixing | cement | fine aggregate | coarse aggregate |
| I | 55.0 | 47.0 | 170 | 340 | 824 | 951 |
| II | 47.8 | 46.7 | 170 | 355 | 814 | 951 |
| III | 43.0 | 47.0 | 165 | 384 | 813 | 938 |
| IV | 41.3 | 46.6 | 165 | 400 | 800 | 938 |

### (Materials used)

- Cement:: Normal Portland cement with a density of 3.16 of Taiheiyo Cement Corporation
- Fine aggregate:: Oigawa river sand with a density of 2.59
- Coarse aggregate:: Crushed stones from Ome with a density of 2.65
- Admixtures:: Polycarbonate type water-reducing agent C-1: RHEOBUILD SP8N made by NMB Co., Ltd. Lignosulfonate type water-reducing agent C-2: POZZOLITH No. 70 made by NMB Co., Ltd.
- Slump measurement:: according to JIS A-1101
- Measurement of entrained air:: according to JIS A-1128

**Table 5: Test results**

| | | Mix | Workability-improving agent | | | | Water-reducing agent | | Workability Evaluation | | |
|---|---|---|---|---|---|---|---|---|---|---|---|
| | | No. | Polyalkylene oxide derivative | Hydrocarbon derivative | Mixing ratio | Added amount | Type | Added amount | Slump | Bleeding | Evaluation |
| Example | 1 | III | A-1 | B-1 | 70/30 | 0.2 | C-1 | 1.0 | none | none | A |
| | 2 | III | A-2 | B-1 | 70/30 | 0.2 | C-1 | 1.0 | none | none | A |
| | 3 | III | A-3 | B-1 | 70/30 | 0.2 | C-1 | 1.0 | none | none | A |
| | 4 | III | A-4 | B-1 | 70/30 | 0.2 | C-1 | 1.0 | none | none | A |
| | 5 | III | A-5 | B-1 | 70/30 | 0.2 | C-1 | 1.0 | none | none | A |
| | 6 | III | A-6 | B-1 | 70/30 | 0.2 | C-1 | 1.0 | none | none | A |
| | 7 | III | A-3 | B-2 | 70/30 | 0.2 | C-1 | 1.0 | none | none | A |
| | 8 | III | A-3 | B-3 | 70/30 | 0.2 | C-1 | 1.0 | none | none | A |
| | 9 | III | A-3 | B-4 | 70/30 | 0.2 | C-1 | 1.0 | none | none | A |
| | 10 | III | A-3 | B-5 | 70/30 | 0.2 | C-1 | 1.0 | none | none | A |
| | 11 | III | A-4 | B-2 | 85/15 | 0.2 | C-1 | 1.0 | none | none | A |
| | 12 | III | A-4 | B-2 | 35/75 | 0.2 | C-1 | 1.0 | little | none | B |
| | 13 | I | A-2 | B-4 | 70/30 | 0.2 | C-2 | 1.0 | none | little | B |
| | 14 | III | A-1 | - | 100/0 | 0.2 | C-1 | 1.0 | little | much | C |
| | 15 | III | - | B-1 | 0/100 | 0.2 | C-1 | 1.0 | little | much | C |
| | 16 | III | A-7 | B-1 | 70/30 | 0.2 | C-1 | 1.0 | little | much | C |
| | 17 | III | A-12 | B-2 | 70/30 | 0.2 | C-1 | 1.0 | none | none | A |
| Comparative Example | 1 | III | A-8 | B-1 | 70/30 | 0.2 | C-1 | 1.0 | much | much | D |
| | 2 | III | A-1 | B-6 | 70/30 | 0.2 | C-1 | 1.0 | much | much | D |
| Reference Example | 1 | II | - | - | - | - | C-2 | 1.0 | none | none | - |
| | 2 | IV | - | - | - | - | C-1 | 1.0 | none | none | - |

| | | | | | | | | | | | |
|---|---|---|---|---|---|---|---|---|---|---|---|
| Note: The added amounts given in the Table are expressed as wt% of the cement weight | | | | | | | | | | | |

**Table 6: Test results**

| | | Mix | Workability-improving agent | | | | Water-reducing agent | | Workability Evaluation | | |
|---|---|---|---|---|---|---|---|---|---|---|---|
| | | No. | Polyalkylene oxide derivative | Hydrocarbon derivative | Mixing ratio | Added amount | Type | Added amount | Slump | Bleeding | Evaluation |
| Example | 18 | III | A-9 | B-2 | 70/30 | 0.2 | C-1 | 1.0 | none | none | A |
| | 19 | III | A-10 | B-1 | 70/30 | 0.2 | C-1 | 1.0 | none | none | A |
| | 20 | III | A-11 | B-1 | 70/30 | 0.2 | C-1 | 1.0 | none | none | A |
| | 21 | III | A-9 | - | 100/0 | 0.2 | C-1 | 1.0 | none | none | A |
| | 22 | III | A-10 | B-1 | 100/0 | 0.2 | C-1 | 1.0 | none | none | A |
| | 23 | III | A-11 | B-1 | 100/0 | 0.2 | C-1 | 1.0 | none | none | A |
| | 24 | III | A-10 A-1 | B-2 | 20/50/ 30 | 0.2 | C-1 | 1.0 | none | none | A |

| | | | | | | | | | | | |
|---|---|---|---|---|---|---|---|---|---|---|---|
| Note: The added amounts given in the Table are expressed as wt% of the cement weight | | | | | | | | | | | |

The embodiments of Examples 12, 14, 15 and 17 given in Table 5, as well as the Examples 21, 22 and 23 given in Table 6 do not form part of this invention but represent background art that is useful for understanding this invention.

From Tables 5 and 6 it is clear that the workability-improving effect obtained in the Examples in which, according to the present invention, a workability-improving agent is used while varying the type of polyalkylene oxide derivative (Examples 1-6, 15-16, 19-20, 22-23) and in the Examples in which a workability-improving agent is used while appropriately varying the type and mixing ratio of the polyalkylene oxide derivative and the hydrocarbon derivative (Examples 7-14, 17-18, 21, 24) is identical to the effect obtained in the Examples in which a conventional technique is used (Reference Examples 1-2). Therefore, it is possible to obtain an adequate workability-improving effect even without increasing the unit cement content.

These results show that the workability-improving effect is obtained no matter whether the polyalkylene oxide derivatives and the hydrocarbon derivatives are used on their own or in combination.

It is further confirmed that, when polyalkylene oxide derivatives and hydrocarbon derivatives are used in combination, there are also cases, depending on the combination, in which a sufficient workability-improving effect is obtained, as shown by Comparative Examples 1-2.

The water reducing properties of cement and the workability of the concrete given below are evaluated by visual observation in order to determine the effect of the water-reducing admixture according to the present invention.

### (The water-reducing properties of the water-reducing admixture for cement)

The water-reducing properties are judged by the added amount of water-reducing admixture, as a proportion of the cement weight, required to obtain a slump of 18.0 ± 1.0 cm. The evaluation criteria are as follows:
A: good (the added amount is 1.0 wt% or less of the cement weight)
B: satisfactory (the added amount is between 1.0 and 1.3 wt% of the cement weight)
C: bad (the added amount is 1.3 wt% or more of the cement weight)

### (Workability evaluation)

Workability is evaluated by an experienced technician for cement compositions with equal slump and equal amount of entrained air by the manner in which slump occurs and by determining whether or not bleeding has occurred after the concrete has been left standing for 30 minutes. The criteria used for the evaluation are those given in Table 3.

### (Composition and mixing of concrete)

Concrete with a target slump of 18.0 ± 1.0 cm and a target amount of entrained air of 4.5 ± 0.5 % is designed with the mixtures shown in Table 7 by weighing each material so as to obtain a mixed amount of 80 litres, introducing all the materials into a 100 litre pan-type forced mixer and mixing them for 120 seconds. Workability is determined by observing the water-reducing properties of the water-reducing agent for cement and the workability of the concrete by means of the slump value of the prepared concrete.

**Table 7: Concrete mixtures**

| Water/ cement ratio (%) | Fine aggregate ratio (%) | Unit amount (kg/m³) | | | |
|---|---|---|---|---|---|
| | | water for mixing | cement | fine aggregate | coarse aggregate |
| 43.0 | 47.0 | 165 | 384 | 813 | 938 |

### (Materials used)

- Cement:: Normal Portland cement with a density of 3.16 of Taiheiyo Cement Corporation
- Fine aggregate:: Oigawa river sand with a density of 2.59
- Coarse aggregate:: Crushed stones from Ome with a density of 2.65

**Table 8: Water-reducing admixture for cement**

| Water-reducing admixture for cement | | | | |
|---|---|---|---|---|
| Polyalkylene oxide derivatives | Hydrocarbon derivatives | Water-reducing agent | Mixing ratio | Sample |
| A-3 | B-1 | RHEOBUILD SPBN | 15/15/70 | AD-1 |
| A-3 | B-1 | RHEOBUILD SPBN | 5/5/90 | AD-2 |
| A-3 | B-1 | RHEOBUILD SPBN | 30/30/40 | AD-3 |
| A-2 | B-2 | POZZOLITH No. 70 | 15/15/70 | AD-4 |
| A-2 | B-2 | POZZOLITH No. 70 L | 15/15/70 | AD-5 |
| A-3 | B-1 | MIGHTY 3000 S | 15/15/70 | AD-6 |
| A-3 | B-1 | CHUPOL HP-11 | 15/15/70 | AD-7 |
| A-9 | B-2 | RHEOBUILD | 15/15/70 | AD-8 |
| A-10 | B-1 | RHEOBUILD SPBN | 15/15/70 | AD-9 |
| A-3 | B-1 | RHEOBUILD | 15/15/70 | AD-10 |
| A-9 | B-2 | RHEOBUILD 4000 | 15/15/70 | AD-11 |

- Slump measurement:: according to JIS A-1101
- Measurement of entrained air:: according to JIS A-1128

**Table 9: Test results**

| Example | Water-reducing admixture for cement | | Evaluation of the water-reducing admixture | Workability evaluation | | |
|---|---|---|---|---|---|---|
| | Sample | Added amount | | Slump | Bleeding | Evaluation |
| 25 | AD-1 | 1.0 | A | none | none | A |
| 26 | AD-4 | 1.0 | A | none | none | A |
| 27 | AD-6 | 1.0 | A | none | none | A |
| 28 | AD-7 | 1.0 | A | none | none | A |
| 29 | AD-2 | 1.0 | A | much | none | C |
| 30 | AD-3 | 2.0 | C | little | little | B |
| 31 | AD-5 | 3.0 | C | little | little | B |
| 32 | AD-8 | 1.0 | A | none | none | A |
| 33 | AD-9 | 1.0 | A | none | none | A |
| 34 | AD-10 | 1.0 | A | none | none | A |
| 3 5 | AD-11 | 1.0 | A | none | none | A |

As is shown by Examples 25-35 given in Table 9, a workability-improving effect was confirmed irrespective of which sample was used. Moreover, good results for both the water-reducing properties and for workability were obtained especially in Examples 25-35.

The workability-improving effect for cement compositions resulting from the use of the workability-improving agent and the water-reducing agent for cement according to the present invention has been confirmed by the above results.

By adding the workability-improving agent and the water-reducing aadmixture for cement according to the present invention it has become possible to improve workability, which decreases considerably as a result of the variation of the aggregate used in the quality of particle diameter, particle size distribution etc., i.e. it has become possible to improve workability as desired by a simple and very economic technique unknown in the prior art.

## Claims

1. A water-reducing-admixture, comprising a workability-improving agent for improving the workability of cement compositions comprising polyalkylene oxide derivatives represented by the formula (1):
R¹-(CO)ₐ-(O)_{b}-(AO)ₙ-X (1)
wherein R¹ is a hydrocarbon group of from 3 to 18 carbon atoms or a residual group of a polyamine derivative; a and b are 0 or 1; AO is an alkylene oxide of from 2 to 3 carbon atoms; (AO)ₙ is a homopolymer or a block or random copolymer of alkylene oxide units of from 2 to 3 carbon atoms, n is an integer of from 1 to 30; X is H, methyl, ethyl, propyl, butyl or SO₃Y¹, where Y¹ is H, Na and NH₄; and a hydrocarbon derivative of formula (2):
R²-(CO)_{c}-OH (2)
wherein R² is a aliphatic hydrocarbon group of 8 to 18 carbon atoms, and c is an integer of 0 or 1, and wherein the mixing ratio of the polyalkylene oxide derivatives to the hydrocarbon derivatives is between 90/10 to 50/50, and at least one water-reducing agent selected from the group consisting of lignosulfonate-type water-reducing agents, polycarboxyate-type water-reducing agents, polyol-type water-reducing agents, naphthalenesulfonate-type water-reducing agents and melaminesulfonate-type water-reducing agents.

2. A water-reducing admixture according to claim 1, wherein R¹ is a hydrocarbon group of from 3 to 18 carbon atoms, b is 1 and X is H or SO₃Y¹, where Y¹ is H, Na and NH₄;

3. A water-reducing admixture according to claim 1 wherein the residual group of a polyamine derivative is at least one selected from the group consisting of polyalkylene amine, polyvinylamine, polyallylamine, polyomithine, polylysine and polyarginine.

4. The water-reducing admixture according to claim 3, wherein the polyalkylene amine is at least one selected from the group consisting of polyethyleneimine, poly(dicarboxyalkylene)polyethyleneimine, diethyleneamine, triethylenetetramine, tetraethylenepentamine, pentaethylenehexamine, dipropylenetriamine, tripropylenetetramine, tetrapropylenepentamine and poly(dicarboxyalkylene)polyamideamine.

5. A water-reducing admixture according to any one of claims 1 to 4, wherein the weight proportion of ethylene oxides contained in the polyalkylene oxide derivatives is 12 % or more.

6. A water-reducing admixture according to claim 5 wherein the weight proportion of ethylene oxides contained in the polyalkylene oxide derivatives is 40 % or more.

7. A water-reducing admixture according to one of the claims 1 to 6, wherein the mixing ratio of the workability-improving agent to the water-reducing agent lies between 5/95 and 70/30.

## Patentansprüche

1. Wasserreduzierendes Zusatzmittel, umfassend ein verarbeitungsfähigkeitsverbesserndes Mittel zur Verbesserung der Verarbeitungsfähigkeit von Zementmassen, umfassend Polyalkylenoxidderivate der Formel (1):
R¹-(CO)ₐ-(O)_{b}-(AO)ₙ-X (1)
worin R¹ für eine Kohiensraasserstoffgruppe mit 3 bis 18 Kohlenstoffatomen oder eine Restgruppe eines Polyaminderivats steht; a und b für 0 oder 1 stehen; AO für ein Alkylenoxid mit 2 bis 3 Kohlenstoffatomen steht; (AO)ₙ für ein Homopolymer oder ein blockartig oder statistisch aufgebautes Copolymer von Alkylenoxid-Einheiten mit 2 bis 3 Kohlenstoffatomen steht; n für eine ganze Zahl von 1 bis 30 steht; X für H, Ethyl, Ethyl, Propyl, Butyl oder SO₃Y¹ steht, wobei Y¹ H, Na und NH₄ bedeutet; und ein Kohlenwasserstoffderivat der Formel (2):
R²-(CO)_{c}-OH (2)
worin R² für eine aliphatische Kohlenwasserstoffgruppe mit 8 bis 18 Kohlenstoffatomen steht und c für eine ganze Zahl mit einem Wert von 0 oder 1 steht, und wobei das Mischungsverhältnis von Polyalkylenoxidderivaten zu Kohlenwasserstoffderivaten zwischen 90/10 und 50/50 liegt und mindestens ein Wasserreduktioxasmittel aus der Gruppe bestehend aus Wasserreduktionsmitteln vom Lignosulfonat-Typ, Wasserreduktionsmitteln vom Polycarboxylat-Typ, Wasserreduktionsmitteln vom Polyol-Typ, Wasserreduktionsmitteln vom Naphthalinsulfonat-Typ und Wasserreduktionsmitteln vom Melaminsulfonat-Typ ausgewählt ist.

2. Wasserreduzierendes Zusatzmittel nach Anspruch 1, wobei R¹ für eine Kohlerzwasserstoffgruppe mit 3 bis 18 Kohlenstoffatomen steht, b für 1 steht und X für H oder SO₃Y¹ steht, wobei Y¹ H, Na und NH₄ bedeutet.

3. Wasserreduzierendes Zusatzmittel nach Anspruch 1, wobei die Restgruppe eines Polyaminderivats mindestens eine aus der Gruppe bestehend aus Polyalkylenamin, Polyvinylamin, Polyallylamin, Polyornithin, Polylysin und Polyarginin ist.

4. Wasserreduzierendes Zusatzmittel nach Anspruch 3, wobei das Polyalkylenamin mindestens eines aus der Gruppe bestehend aus Polyethylenimin, Poly-(dicarboxyalkylen)polyethylenimin, Diethylenamin, Triethylentetramin, Tetraethylenpentamin, Pentaethylenhexamin, Dipropylentriamin, Tripropylentetramin, Tetrapropylenpentamin und Poly-(dicarboxyalkylen)polyamidamin ist.

5. Wasserreduzierendes Zusatzmittel nach einem der Ansprüche 1 bis 4, wobei der Gewichtsanteil von Ethylenoxiden in den Polyalkylenoxidderivaten 12% oder mehr beträgt.

6. Wasserreduzierendes Zusatzmittel nach Anspruch 5, wobei der Gewichtsanteil von Ethylenoxiden in den Polyalkylenoxidderivaten 40% oder mehr beträgt.

7. Wasserreduzierendes Zusatzmittel nach einem der Ansprüche 1 bis 6, wobei das Mischungsverhältnis von verarbeitungsfähigkeitsverbesserndem Mittel zu Wasserreduktionsmittel zwischen 5/95 und 70/30 liegt.

## Revendications

1. Adjuvant de réduction d'eau, comprenant un agent d'amélioration de la maniabilité, destiné à améliorer à maniabilité de compositions de ciment comprenant des dérivés d'oxyde de polyalkylène représentés par la formule (I) :
R¹-(CO)ₐ-(O)_{b}-(AO)ₙ-X (I)
dans laquelle R¹ est un groupement hydrocarboné de 3 à 18 atomes de carbone ou un groupement résiduel d'un dérivé polyamine ; a et b sont 0 ou 1 ; AO est un oxyde d'alkylène de 2 à 3 atomes de carbone ; (AO)ₙ est un homopolymère ou un copolymère séquencé ou statistique de motifs d'oxyde d'alkylène de 2 à 3 atomes de carbone, n est un entier de 1 à 30 ; X est H, méthyle, éthyle, propyle, butyle ou SO₃Y¹ , où Y¹ est H, Na et NH₄ ; et un dérivé hydrocarboné de formule (2) :
R²-(CO)_{c}-OH (2)
dans laquelle R² est un groupement hydrocarboné aliphatique de 8 à 18 atomes de carbone, et c est un entier de 0 ou 1, et où le rapport de mélange entre les dérivés d'oxyde de polyalkylène et les dérivés hydrocarbonés est compris entre 90/10 et 50/50, et au moins un agent de réduction d'eau choisi dans le groupe constitué d'agents de réduction d'eau de type lignosulfonate, d'agents de réduction d'eau de type polycarboxylate, d'agents de réduction d'eau de type polyol, d'agents de réduction d'eau de type naphtalènesulfonate et d'agents de réduction d'eau de type mélaminesulfonate.

2. Adjuvant de réduction d'eau selon à revendication 1, **caractérisé en ce que** R¹ est un groupement hydrocarboné de 3 à 18 atomes de carbone, b est 1 et X est H ou SO₃Y¹, où Y¹ est H, Na et NH₄.

3. Adjuvant de réduction d'eau selon la revendication 1, **caractérisé en ce que** le groupement résiduel d'un dérivé polyamine est au moins un groupement choisi parmi les groupements constitués de polyalkylène-amine, polyvinylamine, polyallylamine, polyornithine, polylysine et polyarginine.

4. Adjuvant de réduction d'eau selon la revendication 3, **caractérisé en ce que** la polyalkylène-amine est au moins un groupement choisi parmi les groupements constitués de polyéthylèneimine, poly(dicarboxyalkylène)polyéthylèneimine, diéthylèneamine, triéthylènetétramine, tétraéthylènepentamine, pentaéthylènehexamine, dipropylènetriamine, tripropylènetétramine, tétrapropylènepentamine et poly(dicarboxyalkylène)polyamideamine.

5. Adjuvant de réduction d'eau selon l'une quelconque des revendications 1 à 4, **caractérisé en ce que** la proportion en poids d'oxydes d'éthylène contenus dans les dérivés d'oxyde de polyalkylène est de 12 % ou plus.

6. Adjuvant de réduction d'eau selon la revendication 5, **caractérisé en ce que** la proportion en poids d'oxydes d'éthylène contenus dans les dérivés d'oxyde de polyalkylène est de 40 % ou plus.

7. Adjuvant de réduction d'eau selon l'une des revendications 1 à 6, **caractérisé en ce que** le rapport de mélange entre l'agent d'amélioration de la maniabilité et l'agent de réduction d'eau est compris entre 5/95 et 70/30.
